(19) Europäisches Patentamt
European Patent Office
Office européen des brevets

(11) **EP 3 217 392 B1**

(12) **EUROPÄISCHE PATENTSCHRIFT**

(45) Veröffentlichungstag und Bekanntmachung des Hinweises auf die Patenterteilung:
**24.02.2021 Patentblatt 2021/08**

(51) Int Cl.:
**G10K 15/00** *(2006.01)*   **G10K 11/18** *(2006.01)*
**G01H 3/08** *(2006.01)*   **G10L 21/00** *(2013.01)*

(21) Anmeldenummer: **17160337.6**

(22) Anmeldetag: **10.03.2017**

(54) **HÖRBARMACHUNG BREITBANDIGER ULTRASCHALLSIGNALE**

RENDERING WIDEBAND ULTRASONIC SIGNALS AUDIBLE

AUDIBILITÉ DE SIGNAUX ULTRASONORES À LARGE BANDE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **11.03.2016 DE 102016104533**

(43) Veröffentlichungstag der Anmeldung:
**13.09.2017 Patentblatt 2017/37**

(73) Patentinhaber: **Sonotec Ultraschallsensorik Halle GmbH**
**06112 Halle / Saale (DE)**

(72) Erfinder:
  • **HOLSTEIN, Peter**
    **04425 Taucha (DE)**
  • **UZIEL, Sebastian**
    **98693 Ilmenau (DE)**
  • **JANUSZKO, David**
    **DE/96450 Coburg (DE)**
  • **THARANDT, Andreas**
    **04205 Leipzig (DE)**
  • **JOHN, Ronald**
    **06124 Halle (Saale) (DE)**
  • **BADER, Nicki**
    **06120 Halle (Saale) (DE)**

(74) Vertreter: **Maikowski & Ninnemann Patentanwälte Partnerschaft mbB**
**Postfach 15 09 20**
**10671 Berlin (DE)**

(56) Entgegenhaltungen:
**WO-A1-2006/025798   JP-A- 2010 002 367**

• **Pettersson: "Ultrasound detector D 230", , 24. November 2014 (2014-11-24), XP055389960, Gefunden im Internet: URL:http://www.batsound.com/inc/files/pdf/ D230UsersManual.pdf [gefunden am 2017-07-11]**
• **Volker Runkel: "Vergleich gängiger Hardware für die Fledermauserfassung", , 26. Oktober 2010 (2010-10-26), XP055389961, Gefunden im Internet: URL:https://web.archive.org/web/2010102622 3231/http://www.ecoobs.de:80/cnt-vergleich -hardware.html [gefunden am 2017-07-11]**
• **BOZENA SMAGOWSKA: "Ultrasonic Noise Sources in a Work Environment", ARCHIVES OF ACOUSTICS, Bd. 38, Nr. 2, 1. Januar 2013 (2013-01-01) , Seiten 169-176, XP055390259, DOI: 10.2478/aoa-2013-0019**
• **Österreichisches Normungsinstitut: "Schallschutz und Raumakustik im Hochbau Teil 2: Anforderungen an den Schallschutz", , 1. Dezember 2002 (2002-12-01), XP055389964, Gefunden im Internet: URL:https://shop.austrian-standards.at/Pre view.action;jsessionid=063B43042106FB385B5 18A3AAF4005CF?preview=&dokkey=103374&se lec tedLocale=de [gefunden am 2017-07-11]**

EP 3 217 392 B1

## Beschreibung

### Gebiet der Erfindung

Die Erfindung betrifft ein Verfahren zur Hörbarmachung von Ultraschallsignalen gemäß Anspruch 1.

Der Amplitudenverlauf des im Zeitbereich aufgenommenen Ultraschallsignals bleibt unverändert. Die Transformation vom Ultraschallbereich in den hörbaren Bereich ist mit Hilfe der vorliegenden Erfindung bis zu einem Faktor von 32 möglich.

### Stand der Technik

Ultraschall wird zunehmend zur technischen Diagnose verwendet. Der Ultraschall kann dabei auf verschiedenste Weise erzeugt werden. Mechanische Reibung, impulsförmige Anregungen Materialdeformationen, Strömungsturbulenzen, Rissvorgänge und elektrische Entladungen erzeugen Ultraschall. Dieser Ultraschall kann als Quelle von Information über das erzeugende Medium angesehen werden. Im industriellen Bereich wird daher Ultraschall beispielsweise in der Instandhaltung (Maschinendiagnose, Suchen von Druckluftleckagen, Finden von elektrischen Entladungen und anderen) verwendet. Ultraschall kann auch gezielt mit geeigneten Quellen (Lautsprecher, Piezoelektrische Quellen, Galtonpfeifen u. a.) erzeugt werden. Diese kommen überall dort zum Einsatz, wo eine deterministische Quelle benötigt wird. Ein Beispiel ist die Überprüfung der Dichtheit von Kabinen oder anderen Volumina bzw. die generelle Fragestellung nach der akustischen Dichtheit.

Eine weitere Art von Ultraschallquellen findet sich im biologischen Bereich. Fledertiere (Ordnung Chiroptera) und andere erzeugen aktiv Ultraschall um sich im Raum zu orientieren oder Objekte zu orten. Die Erfindung lässt sich auch auf den biologischen Bereich anwenden.

Das menschliche Gehör erlaubt die gleichzeitige Erkennung hoher und tiefer Töne im Klangbild. Daraus ergibt sich die Fähigkeit zur akustischen Orientierung, Kommunikation und Gefahrenerkennung. Dies ist auch die Grundlage für Sprache und das Hören und Antizipieren von Musik. Diese Fähigkeit des Menschen kann genutzt werden, um die Eigenschaften einer Schallquelle zu beurteilen. Dies ist für Schallquellen deren Frequenzen im Ultraschallbereich (Frequenzen von ungefähr 16 kHz bis 1 GHz) liegen nicht möglich, da Ultraschall vom menschlichen Gehör nicht wahrgenommen werden kann. Eine Bewertung von Ultraschallsignalen durch das menschliche Gehör und damit auf der Grundlage der Erfahrung von geschulten Mitarbeitern ist von großer technische Bedeutung, da so eine einfache Bewertung der Charakteristik von Schallquellen vorgenommen werden kann. Auf diese Art ist es wäre es möglich eine Übersicht über das Ultraschall-Spektrum zu erhalten.

Es ist demnach von großem technischem Interesse Ultraschallsignale in hörbare Frequenzen zu überführen. Dabei muss jedoch die Charakteristik des Ultraschallsignals erhalten bleiben, um die Auswertung der Signale nicht zu verfälschen. Gängige Verfahren zur Hörbarmachung vereinfachen aber den Signalinhalt von Ultraschallsignalen so stark, dass der spektrale Inhalt des Originalsignals und von dessen Modulation verloren geht. Deshalb können diese Verfahren nur in einfachen Fällen angewendet werden und liefern nur begrenzte und nicht immer korrekte Aussagen.

JP 2010 002367 offenbart ein Signalmesssystem umfassend ein Signaleingabeteil mit einem Sensor zum Aufnehmen von zeitseriellen Analogsignalen, einen A/D-Wandlungsteil zum Wandeln eines analogen Signaleingangs durch das Signaleingangsteil in ein digitales Signal, ein Signalverarbeitungsteil zum Wandeln einer Grundfrequenz (Pitch) des von einem Datenumwandlungsteil übertragenen digitalen Signals, ein DA-Umwandlungsteil zum Umwandeln eines von dem Signalverarbeitungsteil ausgegebenen Signals in ein analoges Signal und ein akustisches Ausgabeteil zum Umwandeln des von dem DA-Umwandlungsteil ausgegebenen analogen Signals in ein akustisches Signal.

WO 2006/025798 A1 betrifft ein System zur akustischen Echtzeitüberwachung von akustischen Signalen. Das Verfahren umfasst das Teilen des akustischen Signals in Segmente einer ausgewählten Dauer; Ableiten eines Zwischensignals, das eine Verkettung von Abschnitten umfasst, wobei jeder Abschnitt für ein entsprechendes Segment repräsentativ ist und eine andere Dauer als das entsprechende Segment aufweist; und Abtasten des Zwischensignals, um ein akustisches Ausgangssignal derart abzuleiten, dass Teile des akustischen Ausgangssignals, das von jeweiligen Abschnitten des Zwischensignals abgetastet wird, die ausgewählte Dauer haben.

In den technischen Spezifikationen des Ultraschalldetektors D230 der Firma Pettersson Elektronik AB wird ein Verfahren zur Hörbarmachung von Ultraschallsignalen beschrieben.

In der Veröffentlichung "Vergleich gängiger akustischer Nachweisverfahren" von V. Runkel vom 26.10.2010 werden Techniken zur Fledermaus-Detektion beschrieben.

Diese Nachteile des Stands der Technik zu beseitigen ist die Aufgabe der vorliegenden Erfindung. Es wird ein Verfahren zur Verfügung gestellt, das eine Transformation eines Ultraschallsignals in den hörbaren Bereich erlaubt ohne dass dessen zeitliche Dynamik verloren geht.

### Zusammenfassung der Erfindung

Die Erfindung betrifft ein Verfahren zur Hörbarmachung von Ultraschallsignalen gemäß Anspruch 1. Das heißt, der Amplitudenverlauf des im Zeitbereich aufgenommenen Ultraschallsignals bleibt unverändert.

Schall und damit auch Ultraschall breitet sich als fortlaufende Störung der lokalen Dichte der Luft im Raum aus. Diese Änderung der lokalen Dichte kann in Form des Schalldrucks p in Abhängigkeit von der Zeit gemessen werden. Eine weitere Schallfeldgröße, die ein Schallfeld beschreibt ist der Schalldruckpegel. Der Schalldruckpegel wird wie folgt

$$L_p = 10 \, lg \left( \frac{(p(t)^2)}{p_0^2} \right)$$

mit $p_0$ als einem geeigneten Bezugswert berechnet. Üblicherweise wird hierfür in der Akustik $p_0 = 20 \, \mu Pa$ genutzt. Das im Zeitraum aufgenommene Signal kann durch eine Fouriertransformation in den Frequenzraum überführt werden.

Für den Menschen übernimmt das Trommelfell des Ohres die Funktion eines Schalldetektors. Der Mensch ist jedoch nur in der Lage in einem begrenzten Bereich von Frequenz und Schalldruckpegel Geräusche wahrzunehmen. Dieser Bereich wird durch das sogenannte Hörfeld definiert. Für den Menschen ist es demnach nur möglich Amplitudenänderungen im Zeitsignal wahrzunehmen, die sich innerhalb des Hörfelds des Menschen befinden. Das Hörfeld des Menschen soll im Rahmen dieser Anmeldung den hörbaren Bereich definieren.

In dem erfindungsgemäßen Verfahren werden bevorzugt nur solche Anteile des Ultraschallsignals verarbeitet, deren Amplitudenvariation im hörbaren Bereich des Menschen liegt. Amplitudenvariationen, die im nichthörbaren des Menschen liegen, werden hingegen bevorzugt nicht betrachtet.

Das erfindungsgemäße Verfahren ermöglicht es daher, dass Ultraschallsignale, die von einem technischen oder biologischen Prozess verursacht werden im für den Menschen hörbaren Bereich dargestellt werden. Die Transformation der Ultraschallsignale in den hörbaren Bereich kann dabei im Wesentlichen in Echtzeit vorgenommen werden. So ist eine Widergabe der Ultraschallsignale im hörbaren Bereich im Wesentlichen synchron zu dem die Ultraschallsignale verursachenden Prozess möglich.

Die Ultraschallsignale werden durch geeignete Mikrofone oder Sensoren aufgenommen und in elektrische Signale umgewandelt. Dadurch bleibt die gesamte physikalische Information, die im akustischen Signal enthalten ist für die weitere Verarbeitung erhalten. Die Signale werden digitalisiert und durch geeignete Transformationen so bearbeitet, dass sie nach der Bearbeitung in einem Frequenzbereich liegen, der das Anhören durch einen Lautsprecher oder Kopfhörer ermöglicht. Nach Durchlauf der gesamten Signalverarbeitungskette liegen die Daten im Zeitbereich vor und können wie gewöhnliche akustische Signale hörbar gemacht werden. Das Zeitsignal kann für Weiterverarbeitungen wie die Berechnung von Pegelwerten, Spektren, Wavelets, Einhüllenden, Spektrogrammen, Kurtogrammen u.a. genutzt werden.

## Detaillierte Beschreibung der Erfindung

In einer bevorzugten Ausführungsform des erfindungsgemäßen Verfahrens werden die Ultraschallsignale bezüglich der Frequenzen komprimiert. Das heißt mit Hilfe des erfindungsgemäßen Verfahrens werden komprimierte akustische Daten erzeugt. Durch die Komprimierung der akustischen Daten, werden diese in den hörbaren Bereich transformiert. Das heißt, es findet eine Komprimierung bezüglich der Frequenz des Signals statt. Durch eine geeignete Wahl des Kompressionsfaktors wird sichergestellt, dass der Amplitudenverlauf erhalten bleibt. Der Kompressionsfaktor wird so gewählt, dass der zu betrachtende Spektralbereich komplett in den Hörbereich fällt. Ein Ausführungsbeispiel für den Ultraschallbereich von 20 bis 100 kHz ist die Wahl eines Kompressionsfaktors von 32.

In einer Ausführungsform des erfindungsgemäßen Verfahrens, werden die hörbar gemachten akustischen Signale nicht wiedergegeben sondern gespeichert. In diesem Fall reduziert die Komprimierung der akustischen Daten die Speichermenge im Vergleich zu den originalen akustischen Daten wesentlich. Dies ist insbesondere bei der Langzeitaufzeichnung von akustischen Daten besonders nützlich.

Erfindungsgemäß wird das Ultraschallsignal mit Hilfe eines geeigneten Mikrophons wie zum Beispiel einem breitbandigen Ultraschallmikrofon oder einem geeigneten Sensor wie zum Beispiel einem breitbandigen Körperschallsensor detektiert.

In einer Ausführungsform des erfindungsgemäßen Verfahrens wird

- das Ultraschallsignal über ein geeignetes Mikrophon oder einen geeigneten Sensor detektiert,
- das detektierte Ultraschallsignal über einen A/D-Wandler in das digitale Signal umgewandelt,
- das digitale Signal an die Recheneinheit übergeben, dies kann zum Beispiel ein Field Programmable Gate Array (FPGA) sein
- ein kontinuierlicher Datenstrom von der Recheneinheit an einen D/A-Wandler übergeben.

Das Ultraschallsignal wird mit einer geeigneten Abtastrate (z. B. 250 kHz) kontinuierlich abgetastet. Das analoge Signal wird in einem Analog/Digital-Wandler (A/D-Wandler) in ein digitales Signal umgewandelt. Dabei werden akustische Anteile des detektierten Signals die im hörbaren Bereich liegen durch einen Hochpass-Filter aus dem Signal gefiltert (z.B. mit einer Grenzfrequenz von 20 kHz). Ein Anti-Alias-Filter sorgt dafür, dass keine höheren Störfrequenzen in das hörbar zu machende Signal zurückgefaltet werden. Für das erfindungsgemäße Verfahren sind zum Beispiel A/D-Wandler mit einer Auflösung von 16 oder 24 bit geeignet.

Das Signal wird anschließend an eine Recheneinheit übergeben. Die Recheneinheit realisiert eine blockweise Signalzerlegung anschließend wird blockweise eine Transformation des Zeitsignals in den Frequenzraum durchgeführt. Diese Transformation in den Frequenzraum wird mit Hilfe einer Fouriertransformation umgesetzt. Anschließend findet eine Rücktransformation des Frequenzsignals in ein Zeitsignal mit Hilfe einer Fourierrücktransformation statt. Anschließend wird eine Synthese der blockweise transformierten Zeitsignale zu einem Zeitsignal durchgeführt. Das neue Zeitsignal verfügt über eine geringere Abtastrate im Vergleich zum Ausgangssignal. Das digitale transformierte Zeitsignal wird in Form eines kontinuierlichen Datenstroms mit geringerer Abtastrate als das Originalsignal an einen Digital-Analog-Wandler (D/A-Wandler) übergeben, der das digitale Zeitsignal in ein analoges Zeitsignal umwandelt. Das transformierte akustische Signal kann nun ausgegeben werden.

In einer Ausführungsform der Erfindung wird ein Field Programmable Gate Array (FPGA) als Recheneinheit verwendet. Ein FPGA ist ein integrierter Schaltkreis der Digitaltechnik in den logische Schaltungen programmiert werden können.

In einer bevorzugten Ausführungsform des erfindungsgemäßen Verfahrens beträgt die Abtastrate des Zeitsignals des transformierten Ultraschallsignals 8 kHz. Diese Bandbreite entspricht dem empfindlichsten Hörbereich des Menschen und ist damit gut wahrnehmbar. Für die Reproduzierung der meisten physikalischen technischen und biologischen Vorgänge ist diese Bandbreite ausreichend. Ebenso ist diese Bandbreite ausreichend, um den Anforderungen an die Reproduzierbarkeit gerecht zu werden.

Durch diese Ausführungsform des erfindungsgemäßen Verfahrens wird eine Frequenzkompression des gesamten spektralen Inhalts des Ultraschallsignals vorgenommen. Das Spektrum wird qualitativ nicht verändert, die spektrale Auflösung verringert sich jedoch um den Kompressionsfaktor. Die zeitliche Modulation eines Ultraschallsignals bleibt auch nach der Anwendung des erfindungsgemäßen Verfahrens exakt erhalten (Erhaltung des Zeitverlaufs der Signaldynamik). Der Frequenzinhalt des Ultraschallsignals wird in den hörbaren Bereich transformiert. Zu beachten ist, dass Frequenzdifferenzen nicht erhalten bleiben sondern ebenfalls der Skalierung mit dem Frequenzfaktor unterliegen. Dies kann bei der Anregung eines Ultraschallsignals mit aktiven Sendern, nachteilig sein, da geringe Modulationsfrequenzen eines Trägersignals (üblicherweise in der Größenordnung von ca. 1 kHz) ebenfalls skaliert werden und dadurch schlechter vom Gehör wahrgenommen werden.

Dieses Verfahren ist insbesondere nützlich, wenn das Ultraschallsignal über einen breiten Frequenzbereich verteilt ist und wenn spektrale Merkmale existieren. Dies ist bei fast allen Ultraschallsignalen, die technisch oder biologisch erzeugt werden der Fall.

Aufgrund des technologischen Konzepts wurde die Frequenzkompression unter Beibehaltung der zeitlichen Dynamik in Form eines Vocoderverfahrens umgesetzt. Da beim Ultraschallbereich in den meisten Fällen keine hoch aufgelösten Spektrallinien erwartet werden, wird das erfindungsgemäße Verfahren in einer Ausführungsform neben der Hörbarmachung auch dazu genutzt, eine erhebliche Datenkompression zur Speicherung und Weiterverarbeitungsalgorithmen zur Verfügung zu stellen. Dies bietet vor allem große Vorteile bei der Langzeitbeobachtung technischer Prozesse. Mit dem erfindungsgemäßen Verfahren wird eine Komprimierung der Daten bis zu einem Faktor von 32 erreicht und hat damit einen ähnlichen Effekt wie die MP3 Komprimierung. Dies wirkt sich positiv auf die effektive Nutzung von Speicherplatz und Rechenleistung aus. Da es im Gegensatz zum Audiobereich keine Normanforderungen bezüglich von Filter- oder Randeffekten (Informationsverlust) gibt, steht der Anwendung Kompression (neben dem Ziel der Hörbarmachung) nichts entgegen. Die Datenkomprimierung erlaubt eine effektive Reduktion des Speicherbedarfs. Das Verfahren ist vor allem dann verlustfrei, wenn das Ultraschallsignal wenig Information enthält. Die ist in den meisten natürlich und technisch erzeugten Ultraschallsignalen der Fall.

Frequenzverschiebungen werden bereits technisch genutzt. Im Audiobereich können beispielsweise Stimmlagen verändert werden. Aus einer Männerstimme kann eine Frauenstimme gemacht werden. Anwendungen finden sich in der Unterhaltungsindustrie usw. Diese Frequenzverschiebungen unter Erhaltung der Zeitmodulation sind aber relativ gering (typischerweise Faktor 1,5 ... 2). Für die Transformation von Ultraschallsignalen sind solch geringe Faktoren nicht geeignet. Die Anwendung des erfindungsgemäßen Verfahrens hat überraschenderweise gezeigt, dass eine Frequenzverschiebung des Ultraschallsignals bis zu einem Faktor von 32 durchgeführt werden kann.

In einer Ausführungsform des erfindungsgemäßen Verfahrens wird die Ausgabe von Signalen genutzt, die nur wenig Information enthalten. Dabei wird das Signal mit Filterbänken in Frequenzbänder zerlegt. Die Frequenzbänder werden dann durch einen entsprechenden rms-Wert (root mean square) repräsentiert.

Das Ultraschallsignal wird digital abgetastet und anschließend werden vom Originalsignal 1/n Oktaven berechnet. Das heißt, das Originalsignal wird in Frequenzbänder zerlegt. Dafür wird eine digitale Filterbank genutzt. Für jede Schmalbandoktave, d.h. für jedes Frequenzband wird ein zeitabhängiger Pegelwert bzw. rms-Wert berechnet. Dieser Pegelwert repräsentiert die Amplitude des Ultraschallsignals in dem jeweiligen Frequenzband.

Für jedes Frequenzband wird ein skaliertes Frequenzband definiert, in das die Ultraschallinformation skaliert wird. Für jedes Frequenzband wird jeweils ein bandpassbegrenztes Rauschen im hörbaren Bereich generiert. Das heiß, die einzelnen Frequenzbänder werden durch Schmalbandrauschen repräsentiert. Das Rauschen wird vorzugsweise von einem digitalen Rauschgenerator in Echtzeit berechnet. Dieses Bandrauschen wird mit dem zugehörigen Pegelwert aus dem höheren Frequenzband verrechnet und das entsprechend gewichtete Rauschen wird ausgegeben. Die simultane Ausgabe aller Schmalbandrauschsignale ergibt dann einen Höreindruck des breitbandigen Ultraschallsignals. Die Intensität des Rauschens bestimmt die Lautstärke, die in diesem Frequenzbereich ausgegeben wird. In einer Ausführungsform des erfindungsgemäßen Verfahrens werden 1/12 Oktaven berechnet, das heißt, es generieren 27 Rauschquellen ein akustisches Signal zur transformierten Wiedergabe eines Signal von 20-100 kHz, was im Höreindruck dem stochastischen Charakter von Ultraschallsignalen in den meisten Fällen gerecht wird.

In einer Ausführungsform des erfindungsgemäßen Verfahrens werden die Frequenzbänder mit einem geeigneten Faktor skaliert. Diese Skalierung kann linear oder auch nichtlinear erfolgen. Durch eine nichtlineare Skalierung ist es möglich bestimmte Frequenzen im Signal zu betonen. Dadurch können Alarmfunktionen in dem beim Auftreten bestimmter Frequenzen indem erfindungsgemäßen Verfahren umgesetzt werden. Die Skalierung mit einem geeigneten Faktor findet vor der Multiplikation mit dem Pegelwert statt.

In einer Ausführungsform der vorliegenden Erfindung kann das erzeugte Rauschen Bandpass begrenztes weißes Rauschen oder rosa Rauschen sein.

Diese Ausführungsform der vorliegenden Erfindung bietet ebenfalls den Vorteil, dass zur Speicherung des Signals eine geringe Speicherkapazität notwendig ist. Die Koeffizienten werden gleichzeitig auch für die Berechnung von Pegeln in Frequenzbändern nutzbar (wichtiges Feature in der Maschinendiagnose). Der Startpunkt und die Filterbreite der verwendeten Filterbank kann für die Hörbarmachung geeignet angepasst werden.

In einer nicht zur Erfindung gehörenden Ausführungsform des erfindungsgemäßen Verfahrens ist ein Überblick über das gesamte Ultraschallspektrum nicht notwendig. Stattdessen sollen Details (z.B. Modulationen) genauer erkannt und die restlichen Frequenzen ausgeblendet werden. Dann setzt man andere Verfahren ein, die das interessierende Ultraschall-Frequenzband in den Hörbereich verschieben.

In einer weiteren nicht zur Erfindung gehörenden Ausführungsform des erfindungsgemäßen Verfahrens wird

•  das originale Zeitsignal des Ultraschallsignals erfasst,
•  ein Schmalbandspektrum um eine Trägerfrequenz ein schmalbandiges Signal erfasst,
•  die Trägerfrequenz automatisch oder manuell variiert und
•  das schmalbandige Signal für jede Trägerfrequenz im hörbaren Bereich nachgebildet.

Das Originale Zeitsignal des Ultraschallsignals wird erfasst. Um eine Trägerfrequenz herum wird ein schmalbandiges Signal erfasst und bewertet, die Zeitmodulation des Signals bleibt dabei erhalten. Der Bezug zur Trägerfrequenz geht verloren. Die Frequenzdifferenz bleibt erhalten.

In einer nicht zur Erfindung gehörenden Ausführungsform des erfindungsgemäßen Verfahrens ist keine Komprimierung der Frequenzen notwendig. Die Breite des Signal Bandes um die Trägerfrequenz das erfasst wird, wird ebenfalls skaliert. In einer nicht zur Erfindung gehörenden Ausführungsform des erfindungsgemäßen Verfahrens entsteht eine Bandbreite von 4 kHz bei der Ausgabe.

In einer weiteren nicht zur Erfindung gehörenden Ausführungsform wird eine Komprimierung der Frequenzen vorgenommen, z.B. wenn die interessierenden Modulationen eine Hub >20 kHz umfassen. Das Verfahren kann so modifiziert werden, dass der gesamte Frequenzbereich, den der Ultraschallsensor erfasst, bewertet werden kann. In dieser Ausführungsform stellen die rms-Werte der schmalbandig erfassten Signale eine komprimierte Repräsentation des Originalsignals dar. Der Rauchanteil, der für die Erreichung einer brauchbaren Hörqualität benötigt wird, wird synthetisch erzeugt und benötigt daher keinen Speicherplatz.

Die Trägerfrequenz kann in dem Verfahren automatisch oder manuell variiert werden. Durch die automatische Abtastung kann ein Pseudospektrum erzeugt werden. Durch die Variation der Trägerfrequenz geht die zeitliche Modulation des Signals erst einmal verloren. Durch die Verarbeitung des gespeicherten Originalsignals wird das schmalbandige Signal für jede Trägerfrequenz nachgebildet, indem das Originalsignal mehrfach neu berechnet wird. Für veränderliche Trägerfrequenzen kann dann die zeitliche Modulation des Signals rekonstruiert werden.

In einer bevorzugten Ausführungsform des erfindungsgemäßen Verfahrens wird das transformierte Signal in Echtzeit über ein geeignetes Medium ausgegeben. Ein geeignetes Medium kann zum Beispiel ein Lautsprecher oder ein Kopfhörer sein. In einer weiteren bevorzugten Ausführungsform wird das transformierte Signal auf einem Speichermedium gespeichert.

In einer Ausführungsform des erfindungsgemäßen Verfahrens wird eine A-Bewertung in die Signalkette implementiert werden. Da die Bewertung des physikalischen Inhalts der Ultraschallsignale von Bedeutung ist, muss berücksichtigt werden, dass Signal nach der Frequenztransformation und der Ausgabe im Hörbereich (Kopfhörer, Lautsprecher) der physiologischen und psychologischen Bewertung durch Gehör und Gehirn unterliegen. Mit der A-Bewertung (DIN IEC 45631) wird dies berücksichtigt. Um die reale physikalische Intensität des Signals nachzuempfinden, kann diese Dämpfung bzw. Verstärkung ausgeglichen werden. Eine Realisierungsvariante besteht darin, im Zeitsignal des Ausgabekanals eine inverse A-Bewertung zu implementieren.

Die Erfindung wird nachfolgend anhand von 3 Zeichnungen näher erläutert.

**Figur 1** Darstellung der Leistungsspektrums bei der Anwendung des Verfahrens mit Hilfe der Fouriertransformation.

**Figur 2** Darstellung des Leistungsspektrums bei der Anwendung des Verfahrens mit Hilfe einer Filterbank

**Figur 3** Darstellung des Leistungsspektrums bei der Anwendung des Verfahrens mit Hilfe der Auswertung eines schmalbandigen Signalbereichs um eine Trägerfrequenz.

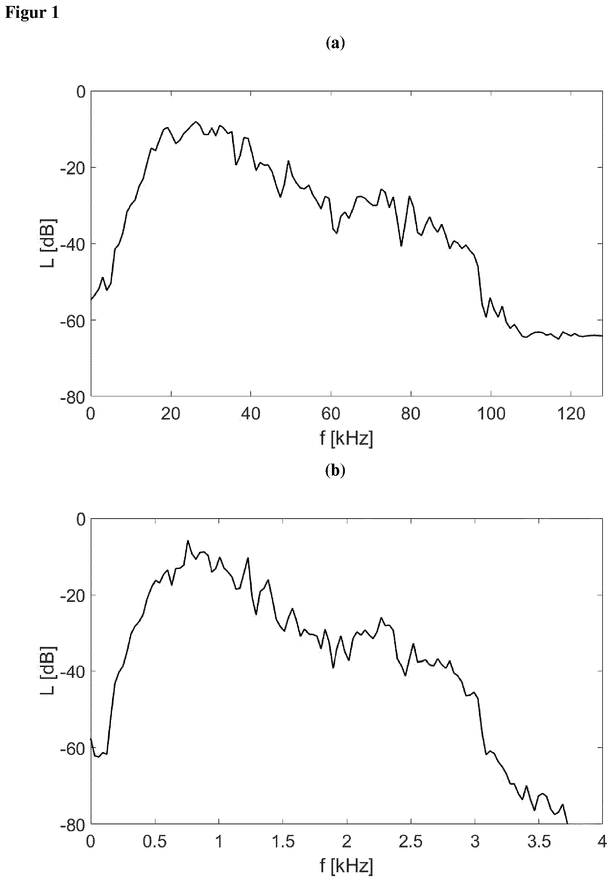

Die Schalldruckwerte in Figur 1 bis Figur 3 sind nicht auf 20 μPa bezogen. Sie stellen nur relative Angaben des Schalldrucks in dB dar.

Die Hörbarmachung bezieht sich auf Signale im Zeitbereich. Die Veranschaulichung erfolgt hier aus Gründen der besseren Verständlichkeit im Frequenzbereich. Die Darstellung im Frequenzbereich veranschaulicht neben der Hörbarmachung auch den Anspruch der Datenkompression. Die Pegelwerte sind nicht auf einen Bezugswert referenziert.

In Figur 1(a) zeigt das Frequenzspektrum (logarithmierte Darstellung des Leistungsspektrums eines realen technisch erzeugten Ultraschallsignals). Die Figur 1(b) stellt das Spektrum dar, das mittels Vocoder-Verfahren und Fouriertransformation berechnet wurde.

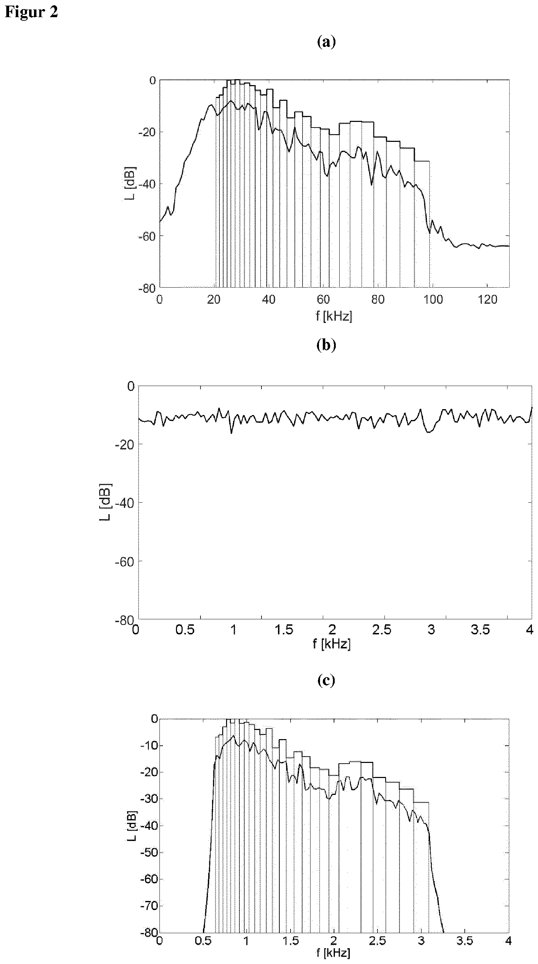

Figur 2 zeigt das Frequenzspektrum (logarithmierte Darstellung des Leistungsspektrums eines realen technisch erzeugten Ultraschallsignals) und die mittels Filterbank erzeugten rms-Werte dieser Bänder. Die Figur 2(b) verdeutlicht die digital erzeugte Rauschkurve, die für die Wichtung mit den Intensitäten der Schmalbandoktaven verwendet wird. Die Figur 2(c) zeigt das Schmalbandoktav-Spektrum, das für die Ausgabe verwendet wird. Die Werte stellen die Intensität des hörbar zu machenden Signals im jeweiligen Band dar, mit dem die Rauschfunktion gewichtet wird.

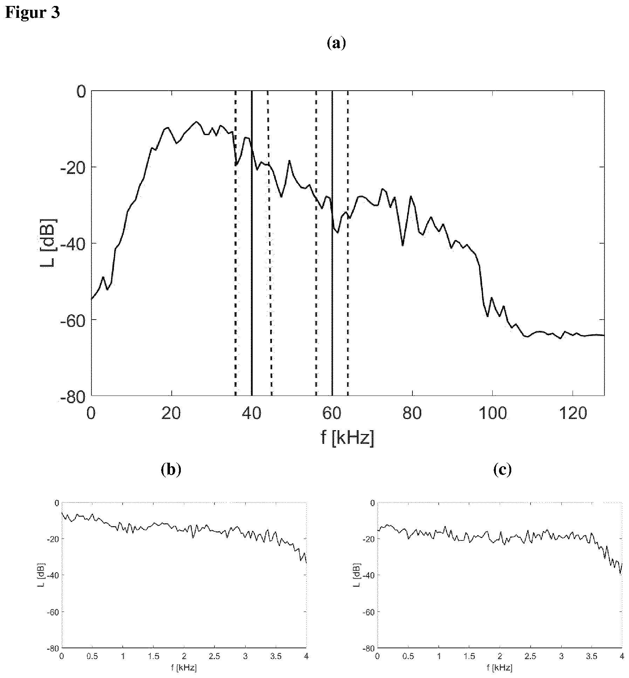

Figur 3(a) zeigt das Frequenzspektrum (logarithmierte Darstellung des Leistungsspektrums eines realen technisch erzeugten Ultraschallsignals). Hervorgehoben ist

der durch die Mischung beeinflusste Teil des Spektrums, der nach der Transformation für die Hörbarmachung zur Verfügung steht. Bei diesem Verfahren wird die Frequenzachse nicht skaliert. Frequenzdifferenzen bleiben erhalten. Die Figuren 3(b) und 3(c) beziehen sich auf die beiden Mischfrequenzen (40 und 60 kHz) und die dadurch bedingte unterschiedliche Intensität bei der jeweiligen Ausgabe. Die niedrigere Intensität im Bereich um 60 kHz im Vergleich zu 40 kHz spiegelt sich auch im Spektrum des heruntergemischten Signals wieder.

**Patentansprüche**

1. Verfahren zur Hörbarmachung von Ultraschallsignalen, wobei ein Ultraschallsignal digital abgetastet wird und wobei die zeitliche Dynamik des Ultraschallsignals erhalten bleibt, **dadurch gekennzeichnet, dass**

- das digital abgetastete Ultraschallsignal an eine Recheneinheit übergeben wird,
- von dem digital abgetasteten Ultraschallsignal 1/n Oktaven durch eine Filterbank berechnet werden,
- für jede 1/n Oktave ein zeitabhängiger Amplitudenpegelwert berechnet wird,
- für jede 1/n Oktave aus dem Ultraschallbereich ein skaliertes Frequenzband im hörbaren Bereich definiert wird, in das die Ultraschallinformation skaliert wird,
- für jedes Frequenzband ein Band-Pass begrenztes Rauschen im hörbaren Bereich generiert wird,
- jedes Rauschen mit dem Amplitudenpegelwert des Frequenzbandes gewichtet wird, und
- ein akustisches Signal durch die simultane Ausgabe aller gewichteten Rauschsignale erhalten wird und das erhaltene akustische Signal ausgegeben wird.

2. Verfahren nach Anspruch 1, **dadurch gekennzeichnet, dass** von dem digital abgetasteten Ultraschallsignal 1/12 Oktaven durch die Filterbank berechnet werden.

3. Verfahren nach Anspruch 1 oder 2, **dadurch gekennzeichnet, dass** nur der Anteil des Ultraschallsignals, dessen Amplitudenvariation im hörbaren Bereich liegt, verarbeitet wird.

4. Verfahren nach einem der Ansprüche 1 bis 3 **dadurch gekennzeichnet, dass** das Ultraschallsignal bezüglich seiner Frequenzen komprimiert wird.

5. Verfahren nach einem der Ansprüche 1 bis 4 **dadurch gekennzeichnet, dass**

- das Ultraschallsignal über ein geeignetes Mikrophon oder einen geeigneten Sensor detektiert wird,
- das detektierte Ultraschallsignal über einen A/D-Wandler in das digitale Signal umgewandelt wird,
- ein kontinuierlicher Datenstrom von der Recheneinheit an einen D/A-Wandler übergeben wird.

6. Verfahren nach Anspruch 5 **dadurch gekennzeichnet, dass** eine Frequenzverschiebung des Ultraschallsignals bis zu einem Faktor von 32 durchgeführt wird.

7. Verfahren nach einem der Ansprüche 2 bis 6, **da-**

**durch gekennzeichnet, dass** die 1/n Oktaven mit einem geeigneten Faktor skaliert werden, wobei die Skalierung linear oder nichtlinear erfolgen kann.

8. Verfahren nach einem der vorangegangenen Ansprüche **dadurch gekennzeichnet, dass** die erhaltenen akustischen Signale in Echtzeit ausgegeben werden.

9. Verfahren nach einem der vorangegangenen Ansprüche **dadurch gekennzeichnet, dass** die erhaltenen akustischen Signale auf ein Speichermedium ausgegeben werden.

10. Verfahren nach einem der vorangegangenen Ansprüche **dadurch gekennzeichnet, dass** bei der Ausgabe der erhaltenen akustischen Signale eine inverse A-Bewertung implementiert wird.

**Claims**

1. Method for making ultrasonic signals audible, wherein an ultrasonic signal is digitally sampled and wherein the temporal dynamics of the ultrasonic signal are maintained, **characterized in that**

   • the digitally sampled ultrasonic signal is transferred to a computing unit,
   • 1/n octaves of the digitally sampled ultrasonic signal are calculated by a filter bank,
   • a time-dependent amplitude level value is calculated for each 1/n octave,
   • for each 1/n octave from the ultrasound range, a scaled frequency band is defined in the audible range, into which the ultrasound information is scaled,
   • for each frequency band, a band-pass limited noise in the audible range is generated,
   • weighting each noise with the amplitude level value of the frequency band, and
   • an acoustic signal is obtained by simultaneously outputting all the weighted noise signals, and the obtained acoustic signal is output.

2. Method according to claim 1, **characterized in that** 1/12 octaves are calculated from the digitally sampled ultrasonic signal by the filter bank.

3. Method according to claim 1 or 2, **characterized in that** only the part of the ultrasonic signal whose amplitude variation is in the audible range is processed.

4. Method according to one of the claims 1 to 3, **characterized in that** the ultrasonic signal is compressed with respect to its frequencies.

5. Method according to one of claims 1 to 4, **charac-**

**terized in that**

   • the ultrasonic signal is detected via a suitable microphone or a suitable sensor,
   • the detected ultrasonic signal is converted into the digital signal via an A/D converter,
   • a continuous data stream is transferred from the computing unit to a D/A converter.

6. Method according to claim 5, **characterized in that** a frequency shift of the ultrasonic signal is carried out up to a factor of 32.

7. Method according to any one of claims 2 to 6, **characterized in that** the 1/n octaves are scaled by a suitable factor, which scaling may be linear or nonlinear.

8. Method according to any one of the preceding claims, **characterized in that** the obtained acoustic signals are output in real time.

9. Method according to any one of the preceding claims, **characterized in that** the obtained acoustic signals are output to a storage medium.

10. A method according to any one of the preceding claims, **characterized in that** inverse A-weighting is implemented in the output of the obtained acoustic signals.

**Revendications**

1. Procédé permettant de rendre audibles des signaux ultrasonores, un signal ultrasonore étant numérisé et la dynamique temporelle du signal ultrasonore étant maintenue, **caractérisé en ce que**

   • le signal ultrasonore numérisé est transféré vers une unité de traitement,
   • 1/n octaves sont calculés à partir du signal ultrasonore numérisé par le biais d'un banc de filtres,
   • une valeur de niveau d'amplitude dépendant du temps est calculée pour chaque 1/n octave,
   • une bande de fréquences cadrée, dans laquelle l'information ultrasonore est cadrée, est définie dans le domaine audible pour chaque 1/n octave du domaine ultrasonore,
   • un bruit limité en passe-bande est généré dans le domaine audible pour chaque bande de fréquences,
   • chaque bruit est pondéré avec la valeur de niveau d'amplitude de la bande de fréquences, et
   • un signal acoustique est obtenu par la délivrance en sortie simultanée de tous les signaux de bruit pondérés et le signal acoustique obtenu est délivré en sortie.

2. Procédé selon la revendication 1, **caractérisé en ce que** 1/12 octaves sont calculés à partir du signal ultrasonore numérisé par le biais du banc de filtres.

3. Procédé selon la revendication 1 ou 2, **caractérisé en ce que** seule la partie du signal ultrasonore dont la variation d'amplitude est dans le domaine audible est traitée.

4. Procédé selon l'une des revendications 1 à 3, **caractérisé en ce que** le signal ultrasonore est comprimé par rapport à ses fréquences.

5. Procédé selon l'une des revendications 1 à 4, **caractérisé en ce que**

   • le signal ultrasonore est détecté par un microphone approprié ou un capteur approprié,
   • le signal ultrasonore détecté est converti en signal numérique par le biais d'un convertisseur A/N.
   • un flux de données continu est transféré de l'unité de traitement vers un convertisseur N/A.

6. Procédé selon la revendication 5, **caractérisé en ce qu'**un décalage de fréquence du signal ultrasonore est effectué jusqu'à un facteur 32.

7. Procédé selon l'une des revendications 2 à 6, **caractérisé en ce que** les 1/n octaves sont cadrés avec un facteur approprié, le cadrage pouvant être linéaire ou non linéaire.

8. Procédé selon l'une des revendications précédentes, **caractérisé en ce que** les signaux acoustiques obtenus sont émis en temps réel.

9. Procédé selon l'une des revendications précédentes, **caractérisé en ce que** les signaux acoustiques obtenus sont délivrés en sortie sur un support de stockage.

10. Procédé selon l'une des revendications précédentes, **caractérisé en ce qu'**une évaluation A inverse est mise en œuvre lors de la délivrance en sortie des signaux acoustiques obtenus.

**Figur 1**

(a)

(b)

**Figur 2**

(a)

(b)

(c)

**Figur 3**

**(a)**

**(b)**                                                      **(c)**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- JP 2010002367 A
- WO 2006025798 A1